(19) Europäisches Patentamt / European Patent Office / Office européen des brevets

(11) **EP 3 558 262 B1**

(12) # EUROPÄISCHE PATENTSCHRIFT

(45) Veröffentlichungstag und Bekanntmachung des Hinweises auf die Patenterteilung:
**09.02.2022 Patentblatt 2022/06**

(21) Anmeldenummer: **17826244.0**

(22) Anmeldetag: **22.12.2017**

(51) Internationale Patentklassifikation (IPC):
***A61K 9/00*** *(2006.01)* ***A61K 36/9066*** *(2006.01)*
***A61K 47/10*** *(2017.01)* ***A61K 47/16*** *(2006.01)*
***A61K 47/42*** *(2017.01)* ***A61K 9/14*** *(2006.01)*
***A61K 9/16*** *(2006.01)* ***A61K 9/19*** *(2006.01)*
***A61K 31/704*** *(2006.01)*

(52) Gemeinsame Patentklassifikation (CPC):
(C-Sets verfügbar)
**A61K 9/146; A61K 9/0019; A61K 9/1658; A61K 9/1694; A61K 9/19; A61K 31/12; A61K 31/136; A61K 31/366; A61K 31/409; A61K 31/473; A61K 31/475; A61K 31/506; A61K 31/704; A61K 47/10; A61K 47/16;** (Forts.)

(86) Internationale Anmeldenummer:
**PCT/EP2017/084522**

(87) Internationale Veröffentlichungsnummer:
**WO 2018/115505 (28.06.2018 Gazette 2018/26)**

(54) **HSA-GALENIK**

HSA GALENICS

GALÉNIQUE HSA

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016 DE 102016125666**

(43) Veröffentlichungstag der Anmeldung:
**30.10.2019 Patentblatt 2019/44**


(73) Patentinhaber:
- **Schrenk, Hans-Hermann 67378 Zeiskam (DE)**
- **Frei, Eva 69124 Heidelberg (DE)**
- **Denck, Petra 60438 Frankfurt am Main (DE)**
- **Denck, Michael 60438 Frankfurt am Main (DE)**

(72) Erfinder: **SCHRENK, Hans-Hermann 60438 Frankfurt am Main (DE)**

(74) Vertreter: **Schüssler, Andrea Kanzlei Huber & Schüssler Truderinger Strasse 246 81825 München (DE)**


(56) Entgegenhaltungen:
**CN-A- 103 054 810** **CN-B- 102 512 404**
**US-A1- 2011 142 948**

- **HOSSEIN MOHAMMAD-BEIGI ET AL: "The Effects of Organic Solvents on the Physicochemical Properties of Human Serum Albumin Nanoparticles", IRANIAN JOURNAL OF BIOTECHNOLOGY, Bd. 14, Nr. 1, 30. März 2016 (2016-03-30), Seiten 45-50, XP55458695, IR ISSN: 1728-3043, DOI: 10.15171/ijb.1168**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 558 262 B1

(52) Gemeinsame Patentklassifikation (CPC): (Forts.)
**A61K 47/42**

C-Sets
**A61K 31/12, A61K 2300/00;**
**A61K 31/136, A61K 2300/00;**
**A61K 31/366, A61K 2300/00;**
**A61K 31/409, A61K 2300/00;**
**A61K 31/473, A61K 2300/00;**
**A61K 31/475, A61K 2300/00;**
**A61K 31/506, A61K 2300/00;**
**A61K 31/704, A61K 2300/00**

## Beschreibung

Die vorliegende Erfindung betrifft die Herstellung von Wirkstoff-Trägerprotein-Lösungen sowie von Wirkstoff-Trägerprotein-Lyophilisaten. Die Wirkstoff-Trägerprotein-Lösungen können insbesondere zur Injektion von an sich wasserunlöslichen Wirkstoffen unter Verwendung des Trägerproteins Serumalbumin, insbesondere von humanem Serumalbumin, als Lösungsvermittler eingesetzt werden.

Bei vielen Wirkstoffen treten aufgrund ihrer oftmals geringen Wasserlöslichkeit Schwierigkeiten bei der intravenösen Verabreichung auf. Oftmals werden Hilfsstoffe zur Formulierung benötigt, wie beispielsweise polyoxyethyliertes Castoröl (Cremophor) oder organische Lösungsmittel, wie beispielsweise Methylenchlorid oder Chloroform, die bei der Verabreichung an einen Patienten toxische Nebenwirkungen hervorrufen können.

Weiterhin sind im Stand der Technik Formulierungen beschrieben, in denen Wirkstoffe zur Verabreichung an Proteine gebunden sind.

DE 196 36 889 A1 beschreibt Transferrin- und AlbuminkKonjugate zytostatischer Verbindungen. Dazu werden thioliertes Transferrin und/oder Albumin durch Maleinimidverbindungen derivatisierten zytostatischen Verbindungen kovalent gekoppelt.

WO 00/71079 A2 beschreibt proteinstabilisierte pharmakologisch aktive Mittel. Dabei werden im Wesentlichen wasserunlösliche Wirkstoffe in Form von suspendierten, mit Protein beschichteten Partikeln bereitgestellt.

DE 198 47 362 A1 beschreibt makromolekulare Wirkstoffkonjugate und ein Verfahren zu ihrer Herstellung. Es erfolgt eine kovalente Kopplung unter Verwendung eines bifunktionellen Säurechlorids.

K. Santhi et al, (Drug Development and Industrial Pharmacy, Vol. 28, No. 9 (2002) 1171-1179) beschreibt Nanopartikel aus Rinderserumalbumin umfassend 5-Fluorouracil. Dabei warden Albuminnanopartikel, die durch Quervernetzung von Albumin unter Verwendung einer Glutaraldehyd-Ethanol-Lösung erhalten werden mit 5-Fluorouracil umgesetzt.

Z. Song et al. (Drug Design, Development and Therapy, 2016:10, 2643-2649) beschreibt mit Curcumin beladene Serumalbumin-Nanopartikel. Die Nanopartikel werden durch Einbringen von humanem Serumalbumin und Curcumin in mit Chloroform gesättigtem Wasser gebildet.

S. Yan et al. (Procedia Engineering 102 (2015) 590-601) beschreibt mit JD27 beladene HSA-Nanopartikel. Zur Herstellung wird eine wässrige HSA-Lösung mit Natronlauge auf pH 9 eingestellt, der Wirkstoff JD27 zugegeben und Nanopartikel durch Zugabe von Glutaraldehyd gebildet.

US 2011/142948 A1 betrifft ein Nanopartikel-Carrier-System basierend auf humanem Serumalbumin für die photodynamische Therapie.

CN 103054810 A1 beschreibt ein Verfahren zur Herstellung einer pharmazeutischen Zusammensetzung enthaltend Curcumin-HSA-Nanopartikel.

CN 102512404 B betrifft in Mizellen eingeschlossenes Curcumin, die eine auf Lunge gerichtete medizinische Präparation darstellen.

Bei den in den oben genannten Dokumenten beschriebenen Vorgehensweisen erfolgt bei der Kopplung eine Denaturierung der Proteine, beispielsweise durch kovalente Kopplung mit dem Wirkstoff oder durch die Bildung von Proteinpartikeln. Es besteht aber die Gefahr, dass solche denaturierten Proteine immunogen wirken. Eine Aufgabe der Erfindung war es deshalb, ein Verfahren bereitzustellen, mit dem schlecht oder nicht wasserlösliche Wirkstoffe, in eine zur Injektion geeignete Formulierung gebracht werden.

Diese Aufgabe wird erfindungsgemäß gelöst durch ein Verfahren zur Herstellung einer Nanopartikel-freien Wirkstoff-Trägerprotein-Lösung umfassend die Schritte:

i) Bereitstellen einer wässrigen Trägerprotein-Lösung, wobei als Trägerprotein Serumalbumin eingesetzt wird,
ii) Auflösen eines schlecht wasserlöslichen oder nicht wasserlöslichen Wirkstoffes in Acetonitril und/oder einem Alkohol ausgewählt aus Methanol, Ethanol, n-Propanol, iso-Propanol und/oder tert-Butanol,
iii) Zugeben der Wirkstoff-Alkohol-Lösung und/oder der Wirkstoff-Acetonitril-Lösung zu der wässrigen Trägerprotein-Lösung zum Erhalten einer Wirkstoff-Trägerprotein-Lösung, wobei das Molverhältnis von Trägerprotein zu Wirkstoff 10:1 bis 1:3 beträgt und wobei die Wirkstoff-Trägerprotein-Lösung keine Partikel mit einem Durchmesser ≥ 50 nm enthält,
iv) Lyophilisieren der in Schritt iii) erhaltenen Lösung und
v) Resuspendieren des Lyophilisats.

Erfindungsgemäß wurde festgestellt, dass unter Verwendung des Trägerproteins Serumalbumin, bevorzugt von humanem Serumalbumin, als Lösungsvermittler, Wirkstoffe, insbesondere wasserunlösliche Wirkstoffe, in eine zur Injektion geeignete Formulierung gebracht werden können.

In einem ersten Schritt wird erfindungsgemäß eine wässrige Trägerprotein-Lösung bereitgestellt. Als Trägerprotein wird Serumalbumin verwendet, insbesondere humanes Serumalbumin. Weiterhin besonders bevorzugt wird als

Trägerprotein rekombinantes humanes Serumalbumin (rHSA) eingesetzt. Besonders bevorzugt wird als Trägerprotein ein Liganden-freies humanes Serumalbumin (liganden-freies HSA) eingesetzt. In einem Liganden-freien humanen Serumalbumin sind die hydrophoben Bindungsstellen nicht besetzt und stehen so zur nicht-kovalenten Bindung mit hydrophoben Wirkstoffen zur Verfügung. Kommerziell erhältliches humanes Serumalbumin aus Humanblut ist oftmals mit Octanoat versetzt, damit hydrophobe Bindungsstellen sicher besetzt sind. Das erfindungsgemäß besonders bevorzugt eingesetzte Liganden-freie HSA ist aber gerade frei von solchen Stoffen wie Octanoat oder Fettsäuren, die die hydrophoben Bindestellen des HSA blockieren. Vorzugsweise wird erfindungsgemäß beim Bereitsstellen der wässrigen Trägerprotein-Lösung ein Schritt durchgeführt, bei dem eventuell vorhandene Liganden, wie beispielsweise Octanoat oder andere Fettsäuren entfernt werden. Vorzugsweise werden solche Liganden, insbesondere Octanoat und andere Fettsäuren durch Filtration über Aktivkohle aus dem HSA Präparat entfernt.

Für Veterinäranwendungen können aber auch tierische Serumalbumine, beispielsweise bovines Serumalbumin eingesetzt werden.

Das Trägerprotein wird erfindungsgemäß in Wasser gelöst, um eine wässrige Trägerprotein-Lösung zu bilden. Diese wässrige Trägerprotein-Lösung enthält vorzugsweise keine weiteren Substanzen neben dem Trägerprotein und dem Wasser und insbesondere keine weiteren Lösungsmittel. Die wässrige Trägerprotein-Lösung besteht somit vorzugsweise aus Trägerprotein und Wasser. Erfindungsgemäß bevorzugt enthält die wässrige Trägerprotein-Lösung, insbesondere HSA-Lösung 1 bis 20% w/v Trägerprotein auf Wasser, mehr bevorzugt 2 bis 10% w/v und noch mehr bevorzugt 4 bis 6% w/v.

In einer weiteren bevorzugten Ausführungsform, insbesondere zur Herstellung von Curcumin-Trägerprotein-Lösungen, wird die in Schritt i) bereitgestellte wässrige Trägerprotein-Lösung auf einen pH-Wert von 5,5 bis 6,5 eingestellt, z.B. mittels eines Citrat-Puffers.

In einem weiteren Schritt wird erfindungsgemäß eine Wirkstofflösung bereitgestellt. Erfindungsgemäß wird dabei als Lösungsmittel ein Alkohol mit einem Siedepunkt von < 100°C eingesetzt oder/und Acetonitril (Siedepunkt: 82°C). Erfindungsgemäße Alkohole zur Bereitstellung der Wirkstofflösung sind Methanol, Ethanol, n-Propanol, iso-Propanol, sec-Butanol und tert-Butanol, bevorzugt Methanol, Ethanol, n-Propanol und iso-Propanol, noch mehr bevorzugt Methanol, Ethanol und iso- Propanol und am meisten bevorzugt Ethanol.

Durch die Verwendung eines oder mehrerer Alkohole und/oder Acetonitril kann erfindungsgemäß die Zugabe von anderen organischen Lösungsmitteln, beispielsweise von halogenierten Lösungsmitteln, Ketonen, Estern oder Ethern vermieden werden. Insbesondere ist erfindungsgemäß die Verwendung von halogenierten und insbesondere chlorierten Lösungsmitteln, wie etwa Methylenchlorid oder Chloroform nicht erforderlich. Auch die Verwendung von anderen Lösungsmitteln, wie etwa polyoxyethyliertem Castoröl ist erfindungsgemäß nicht erforderlich. Bevorzugt wird deshalb erfindungsgemäß als einziges Lösungsmittel ein oder mehrere Alkohole mit einem Siedepunkt von < 100°C und/oder Acetonitril eingesetzt und noch mehr bevorzugt wird als alleiniges Lösungsmittel eine Zusammensetzung, bestehend aus Methanol, Ethanol, iso-Propanol und/oder Acetonitril als Lösungsmittel verwendet. Durch die Verwendung eines Alkohols mit einem Siedepunkt < 100°C und/oder Acetonitril wird im nachfolgenden Lyophilisierungsschritt sichergestellt, dass der Alkohol oder das Acetonitril zusammen mit dem Wasser beim Trocknungsschritt vollständig aus dem Wirkstoff-Trägerproteinprodukt entfernt wird.

Das erfindungsgemäße Verfahren ist grundsätzlich auf alle Wirkstoffe, die in eine zur Injektion geeignete Formulierung gebracht werden sollen, anwendbar. Erfindungsgemäß wird es auf schlecht wasserlösliche oder wasserunlösliche Wirkstoffe angewandt, also auf Wirkstoffe, die eine geringe oder keine Löslichkeit in Wasser aufweisen, insbesondere auf Wirkstoffe, die eine Löslichkeit von weniger als 10 mg/ml, noch mehr bevorzugt weniger als 5 mg/ml und noch mehr bevorzugt weniger als 1 mg/ml in Wasser bei 25°C aufweisen.

In einer bevorzugten Ausführungsform wird als Wirkstoff ein Zytostatikum eingesetzt.

Besonders vorteilhafte Ergebnisse wurden erfindungsgemäß bei Einsatz von Curcumin, Amsacrin, Imatinib, , Mitoxantron, Artemisinin oder Elipticin erhalten. Am meisten bevorzugt wird erfindungsgemäß als Wirkstoff Curcumin, Amsacrin, Mitoxantron oder Artemisinin eingesetzt.

Möglich, aber nicht bevorzugt, ist auch der Einsatz von Doxorubicin.

Weiterhin bevorzugt wird als Wirkstoff ein Farbstoff, insbesondere ein Fluoreszenzfarbstoff eingesetzt. Besonders geeignet sind Farbstoffe für die photodynamische Therapie, wie z.B. meso-Tetrahydrophenylchlorin (mTHPC). Zur photodynamischen Therapie wird zunächst mit dem erfindungsgemäßen Verfahren ein mTHPC-HSA oder ein anderes geeignetes Farbstoff-Trägerprotein-Konjugat hergestellt. Dieses wird dann dem Patienten i.v. verabreicht und reichert sich aufgrund des Trägerproteins, insbesondere HSA, im Zielgewebe an. Zielgewebe ist insbesondere Tumorgewebe, wie z.B. Plattenepithelkarzinom-Gewebe. Anschließend kann eine Fotoaktivierung des Farbstoff-Trägerprotein-Konjugats, insbesondere mTHPC-HSA, durchgeführt werden, beispielsweise durch Bestrahlung mit einem Laser mit definierter Wellenlänge (z.B. 652 nm). Durch die Fotoaktivierung werden im Zielgewebe, insbesondere Tumorgewebe reaktive Spezies gebildet, die das Tumorgewebe zerstören.

Im nächsten Schritt wird erfindungsgemäß die Wirkstoff-Alkohol-Lösung der wässrigen Trägerprotein-Lösung, insbesondere der wässrigen HSA-Lösung (vorzugsweise 5% w/v) zugegeben. Die Zugabe kann beispielsweise durch

langsames Eintropfen unter Rühren erfolgen.

Besonders günstige Ergebnisse werden erhalten, wenn das Molverhältnis von Trägerprotein zu Wirkstoff 10:1 bis 1:3, insbesondere 5:1 bis 1:2, bevorzugt 1:1 bis 1:5 und noch mehr bevorzugt 1:2 bis 1:4 beträgt. Bei einer solchen Vorgehensweise liegen verhältnismäßig wenige Wirkstoffmoleküle pro Trägerproteinmolekül vor und das Trägerprotein bleibt in seiner natürlichen, nicht denaturierten Form.

In einer weiteren bevorzugten Ausführungsform beträgt das Molverhältnis von Trägerprotein zu Wirkstoff 10:1 bis 1:3, bevorzugt 5:1 bis 1:3, mehr bevorzugt 5:1 bis 1:2, insbesondere 2:1 bis 1:2, bevorzugt 1,5:1 bis 1:1,5 und noch mehr bevorzugt 1,2:1 bis 1:1,2. Es wurde festgestellt, dass bei einem äquimolaren Verhältnis von Trägerprotein zu Wirkstoff besonders gute Ergebnisse erhalten werden und das Trägerprotein in natürlicher, nicht denaturierter Form vorliegt. Insbesondere werden bei einem solchen Verhältnis über lange Zeit lagerstabile Lösungen erhalten.

Erfindungsgemäß bevorzugt liegt das Trägerprotein, insbesondere Albumin in der erfindungsgemäß hergestellten Wirkstoff-Trägerprotein-Lösung, insbesondere in einer erfindungsgemäß hergestellten wässrigen Wirkstoff-Trägerprotein-Lösung und in dem darin enthaltenen Wirkstoff-Trägerprotein-Komplex in nativer Form vor.

Weiterhin bevorzugt liegt das Trägerprotein vereinzelt vor, also als Monomer mit daran gekoppeltem Wirkstoff und nicht als Polymer und nicht als Trägerproteinpartikel.

Weiterhin bevorzugt weist die erfindungsgemäß hergestellte Wirkstoff-Trägerprotein-Lösung Wirkstoff-Trägerprotein-Komplexe auf, in denen das Trägerprotein und der Wirkstoff nicht-kovalent aneinander gebunden sind. Bevorzugt sind das Trägerprotein und der Wirkstoff durch hydrophobe Wechselwirkungen aneinander gebunden.

Insbesondere durch die Kombination von geringer Beladung des Trägerproteins mit Wirkstoff, also durch ein Molverhältnis von Trägerprotein zu Wirkstoff von 10:1 bis 1:3, bevorzugt 5:1 bis 1:2 und insbesondere 5:1 bis 1:1,5 und durch die nicht-kovalente Bindung der gering oder nicht-wasserlöslichen Wirkstoffe an das Trägerprotein wird erreicht, dass das Trägerprotein in der erfindungsgemäß hergestellten Wirkstoff-Trägerprotein-Lösung in nativer Form vorliegt. Dies ist wichtig, da denaturiertes Albumin, wie es bei hohen Beladungen mit Wirkstoff gebildet wird, immunogen wirkt.

Die Zugabe der Wirkstoff-Alkohol-Lösung und/oder der Wirkstoff-Acetonitril-Lösung zur wässrigen Trägerprotein-Lösung erfolgt vorzugsweise derart, dass der Anteil des organischen Lösungsmittels in der gebildeten Lösung ≤ 20% v/v, vorzugsweise ≤ 15% v/v ist. Weiterhin beträgt der pH-Wert der Wirkstoff-Trägerprotein-Lösung vorzugsweise einen Wert im Bereich von pH 5,5 bis pH 8, vorzugsweise pH 5,9 bis pH 7,5 oder wird auf einen solchen Wert eingestellt. Auf jeden Fall muss ein pH-Wert von 4,9 vermieden werden, da dies dem isoelektrischen Punkt des Albumins entspricht und dabei das Protein koaguliert.

In einem weiteren Schritt wird dann die aus der Wirkstoff-Alkohol-Lösung bzw. Wirkstoff-Acetonitril-Lösung und der wässrigen Trägerprotein-Lösung gebildete Zusammensetzung lyophilisiert. Vorzugsweise erfolgt die Lyophilisierung durch Einfrieren bei -80°C und Gefriertrocknen. In diesem Verfahrensschritt wird ein gefriergetrocknetes Pulver, enthaltend das Trägerprotein und den Wirkstoff, gebildet. Vorzugsweise wird bei der Lyophilisierung der Alkohol und/oder das Acetonitril und das Wasser vollständig entfernt.

Das gefriergetrocknete Pulver kann als solches gelagert und transportiert werden. Vorzugsweise wird das gefriergetrocknete Pulver erst kurz vor der Anwendung resuspendiert, beispielsweise in einem pharmazeutisch akzeptablen Medium. Bevorzugt erfolgt das Resuspendieren in physiologischer Kochsalzlösung.

Ein weiterer Gegenstand der vorliegenden Erfindung ist deshalb ein Kit umfassend ein Lyophilisat erhältlich mit dem erfindungsgemäßen Verfahren sowie ein pharmazeutisch annehmbares Medium, wie beispielsweise eine physiologische Kochsalzlösung.

Erfindungsgemäß wurde insbesondere festgestellt, dass bei Resuspendieren des hergestellten Lyophilisat eine klare Lösung erhalten wird und nicht eine Suspension. Es wird angenommen, dass unter Verwendung des Trägerproteins Serumalbumin und bevorzugt von humanem Serumalbumin und am meisten bevorzugt von Liganden-freiem HSA als Lösungsvermittler nicht kovalent gebundene Wirkstoff-Trägerproteinkomplexe gebildet werden, in denen das Trägerprotein in nicht denaturierter Form und somit weiterhin in löslicher Form vorliegt. Die im Stand der Technik bei Verwendung von organischen Lösungsmitteln, wie etwa halogenierten organischen Lösungsmitteln beobachtete Coagulierung, beziehungsweise Nanopartikelbildung tritt erfindungsgemäß nicht auf. Deshalb können tatsächlich klare Lösungen und nicht, wie im Stand der Technik oftmals beschrieben, lediglich Suspensionen erhalten werden.

Erfindungsgemäß können somit schlecht oder nicht wasserlösliche Wirkstoffe in einer physiologischen wasserlöslichen Form durch nicht-kovalente Kopplung an natives und insbesondere Liganden-freies HSA als Lösungsvermittler zu Verfügung gestellt werden. Insbesondere sind die erfindungsgemäß hergestellten Wirkstoff-Trägerprotein-Lösungen zur parenteralen Applikation geeignet. Durch die nicht-kovalente Bindung der Wirkstoffe an das Trägerprotein und insbesondere Albumin bleibt das Trägerprotein in nativer löslicher Form. Albumin hat zudem die Eigenschaft sich in Entzündungen und Tumoren anzureichern und führt zudem zu einer deutlich verlängerten Zirkulationszeit des gekoppelten Wirkstoffes.

Von besonderem Vorteil ist die relativ einfache erfindungsgemäße Herstellungsweise, die insbesondere eine reproduzierbare und sichere Herstellung von Trägerprotein-Konjugaten und insbesondere Albumin-Konjugaten mit schlecht oder nicht wasserlöslichen Wirkstoffen ermöglicht.

Insbesondere enthalten die erfindungsgemäßen Wirkstoff-Trägerprotein-Lösungen keine Partikel mit einem Durchmesser von ≥ 50 nm. Die Abwesenheit von Nanopartikeln in den erfindungsgemäß erhältlichen klaren Lösungen kann beispielsweise durch Ultrafiltration überprüft werden. Soweit gewünscht kann die erfindungsgemäße Wirkstoff-Trägerprotein-Lösung vor der Anwendung noch einer Sterilfiltration unterzogen werden.

Die erfindungsgemäßen Wirkstoff-Trägerprotein-Lösungen sind insbesondere für parenterale Anwendungen und am meisten bevorzugt für eine i.v.-Applikation geeignet.

Die Erfindung wird durch die beigefügten Beispiele weiter erläutert.

Beispiel 1

Curcumin nicht kovalent an HSA gebunden

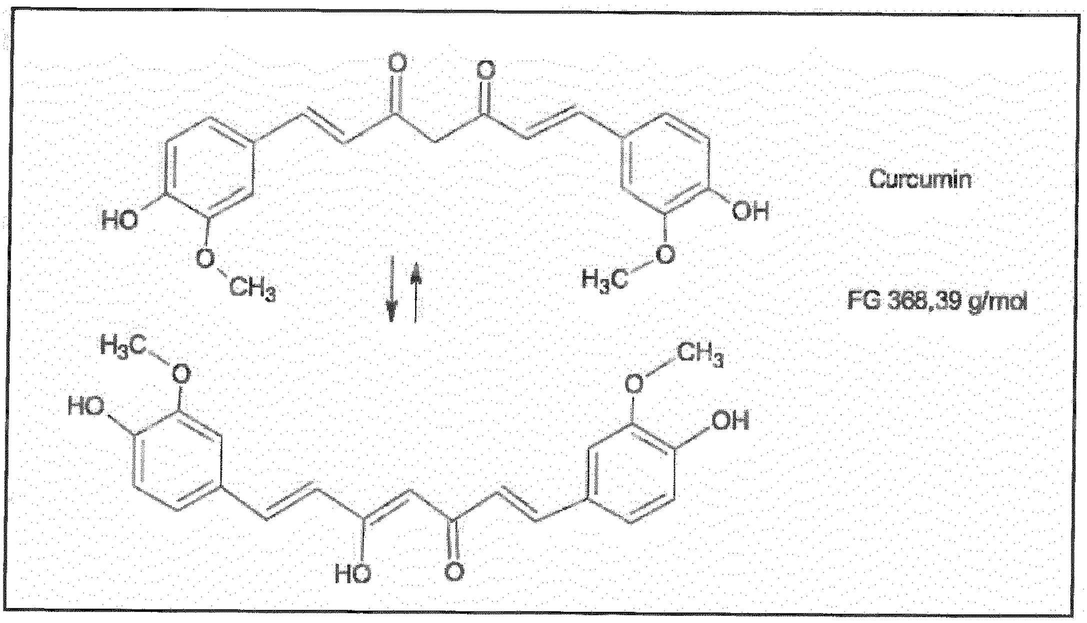


*66000mg/3×369 = 1000mg/X daraus folgt: X = 17mg Curcumin auf 1g HSA.*

Herstellung

1 g HSA wird in 20 ml Wasser (z.B. Ampuwa, also Wasser zur Injektion) gelöst. 17 mg Curcumin werden in 4 ml Ethanol absolut gelöst. Die Ethanol-Lösung wird unter Rühren langsam in die HSA-Lösung tropfen gelassen. Diese Lösung wird bei -80°C eingefroren und gefriergetrocknet. Das Lyophillisat wird mit physiologischer Kochsalzlösung resuspendiert. Zur Kontrolle, dass tatsächlich keine Nanopartikel vorliegen, wird ein Aliquot (2 ml) mit Centricon-Ultrafiltration untersucht. Anschließend erfolgt eine Sterilfiltration der Lösung zur Anwendung.

Beispiel 2

Curcumin nicht kovalent an HSA gebunden

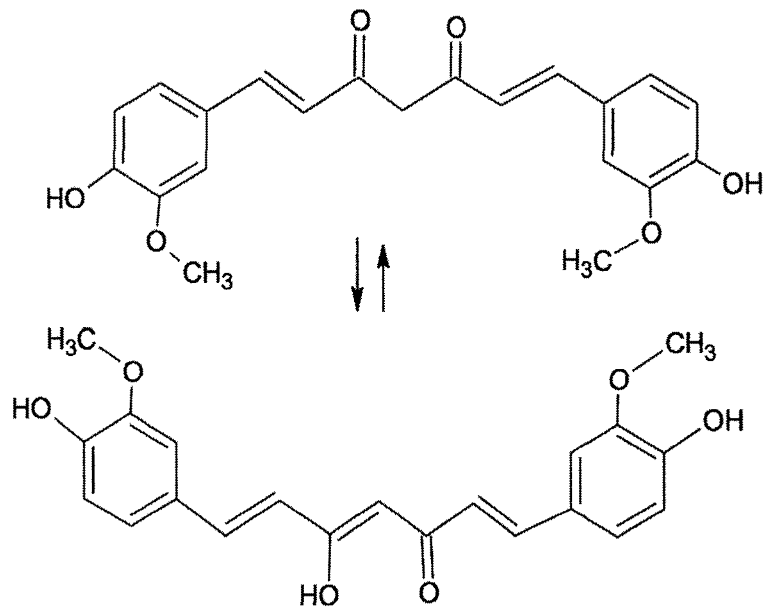

Curcumin

FG 368,39 g/mol

66000mg/369mg = 1000mg/X daraus folgt: X = 5,6mg Curcumin auf 1g HSA.

Herstellung

1g HSA wird in 30ml Ampuwa und 0,5 ml Citratpuffer pH 6,0 (z.B. Fa. Sigma Best. Nr. C9999) gelöst; 5,6mg Curcumin wurden in 3 ml Ethanol abs. unter Erwärmung auf 50°C gelöst. Nach Abkühlen auf Raumtemperatur wird die Curcumin/Ethanol-Lösung unter Rühren langsam in die HSA-Lösung tropfen gelassen. Anschließend wird diese Lösung bei -80° C eingefroren und gefriergetrocknet. Das Lyophillisat wird mit 20 ml physiologischer Kochsalzlösung resuspendiert. Kontrolle eines Aliquotes (2 ml) des Ansatzes mittels Centrion-Ultrafiltration oder GPC-HPLC Anschließend Sterilfiltration der Lösung zur Anwendung.

Beispiel 3

Anwendungsbeispiel: Doxorubicin

***Doxorubicin (FG 579 g/mol)***

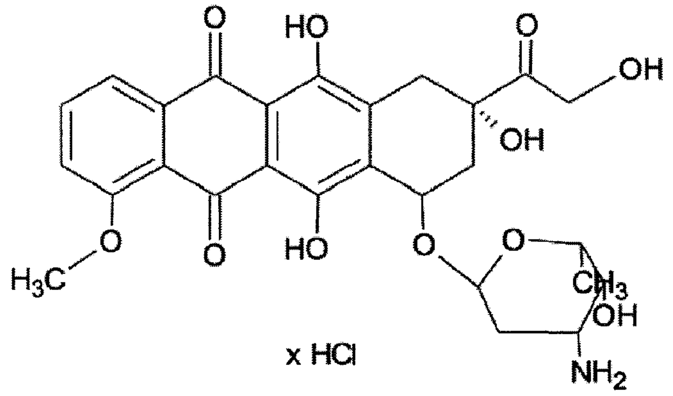

x HCl

Doxorubicin HCl

FG 578,98 g/mol

*66000mg/3×579 = 1000mg/Xdaraus folgt: x=27mg Doxorubicin auf 1g HSA.*

Herstellung Doxorubicin-HSA

1 g HSA wird in 20 ml Wasser (z.B. Ampuwa; d.h. Wasser zur Injektion) gelöst. 27 mg Doxorubicin werden in 4 ml Ethanol absolut gelöst. Die Ethanol-Lösung wird unter Rühren langsam in die HSA-Lösung tropfen gelassen. Diese Lösung wird bei -80°C eingefroren und gefriergetrocknet. Das Lyophillisat wird mit physiologischer Kochsalzlösung resuspendiert. Zur Kontrolle wird ein Aliquot (2 ml) mit Centricon-Ultrafiltration untersucht. Anschließend erfolgt eine Sterilfiltration der Lösung zur Anwendung.

Beispiel 4

Anwendungsbeispiel: Amsacrin-HSA nicht kovalent gebunden

| |
|---|
| Amsacrin ***(FG 393 g/mol)*** 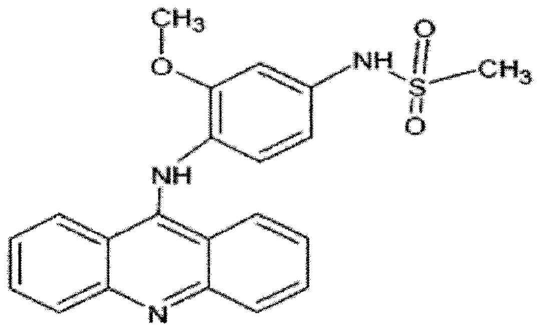 |
| *Diese Beispiel-Substanz hat eine Proteinbindung größer 98% und ist in Ethanol löslich. 6600mg/3×393 = 1000mg/X daraus folgt: X = 18mg Amsacrine auf 1g HSA.* |

Herstellung

1 g HSA wird in 20 ml Wasser (z.B. Ampuwa, d.h. Wasser zur Injektion) gelöst. 18 mg Amsacrin werden in 4 ml Ethanol absolut gelöst. Die Ethanol-Lösung wird unter Rühren langsam in die HSA-Lösung tropfen gelassen. Diese Lösung wird bei -80 °C eingefroren und gefriergetrocknet. Das Lyophillisat wird mit physiologischer Kochsalzlösung resuspendiert. Zur Kontrolle wird ein Aliquot (2 ml) mit Centricon-Ultrafiltration untersucht. Anschließend erfolgt eine Sterilfiltration der Lösung zur Anwendung.

Beispiel 5: Amsacrin nicht kovalent an HSA gebunden

| |
|---|
| Amsacrin (FG 393 g/mol) 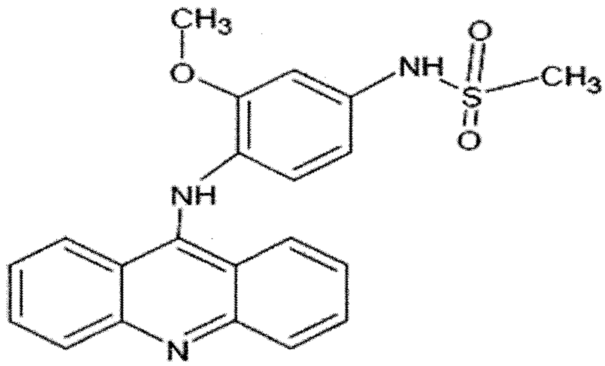 |
| 66000mg/393mg = 1000mg/X daraus folgt : X = 6,0mg Amsacrin auf 1g HSA. |

Herstellung

1g HSA wird in 30ml Ampuwa und 0,5 ml Citratpuffer pH 6,0 gelöst; 6,0mg Amsacrin werden in 3ml Ethanol absolut unter Erwärmung auf 50°C gelöst. Die Ethanol-Lösung wird unter Rühren langsam in die HSA-Lösung tropfen gelassen. Diese Lösung wird bei -80° C eingefroren und gefriergetrocknet. Das Lyophillisat wird mit physiologischer Kochsalzlösung resuspendiert. Kontrolle eines Aliquotes (2ml) des Ansatzes mittels Centricon-Ultrafiltration oder GPC-HPLC. Anschließend Sterilfiltration der Lösung zur Anwendung.

Beispiel 6

Elipticin

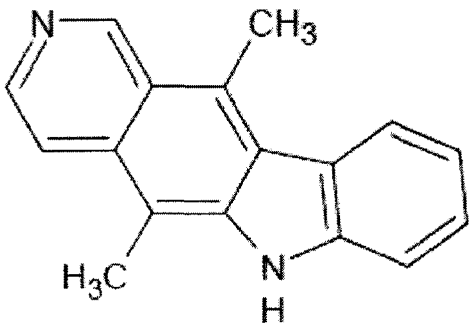

Molekularformel $= C_{17}H_{14}N_2$

Molekulargewicht $= 246.30646$

Berechnung des Ansatzes:

66000 mg / 3x246 mg = 1000 mg/ X mg ;
X = 11,2 mg Elipticin auf 1g HSA (Humanes Serumalbumin)

Herstellung:

1 g HSA in 50 ml Ampuwa lösen; 11,2 mg Mitoxantron in 10 ml Ethanol absolut lösen. Die Ethanol-Lösung unter Rühren langsam in die HSA-Lösung tropfen lassen. Diese Lösung wird bei -80 °C eingefroren und gefriergetrocknet. Das Lyophilisat wird mit physiologischer Kochsalzlösung resuspendiert. Zur Kontrolle wird ein Aliquot (2 ml) mit Centricon-Ultrafiltration untersucht. Anschließend erfolgt eine Sterilfiltration der Lösung zur Anwendung.

Beispiel 7

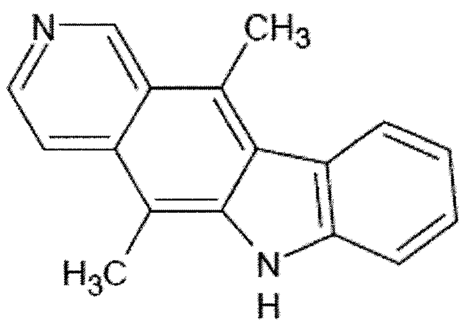

Molekularformel $= C_{17}H_{14}N_2$

Molekulargewicht $= 246.30646$

Elipticin

Berechnung des Ansatzes:

$$66000mg / 246mg = 1000mg / X\ mg ;$$

X = 3,7mg Elipticin auf 1g HSA (Humanes Serumalbumin)

Herstellung:

1g HSA in 50ml Ampuwa lösen; 3,7mg Elipticin in 10ml Ethanol absolut unter Erwärmung auf 50°C lösen. Nach Abkühlung auf Raumtemperatur wird die Ethanol-Lösung unter Rühren langsam in die HSA-Lösung tropfen gelassen. Diese Lösung wird bei -80° C eingefroren und gefriergetrocknet. Das Lyophillisat mit 20ml physiologischer Kochsalzlösung resuspendiert. Anschließend Sterilfiltration der Lösung zur Anwendung. Kontrolle eines Aliquotes (2ml) des Ansatzes mittels Centricon-Ultrafiltration oder GPC-HPLC.

Beispiel 8

Mitoxantron

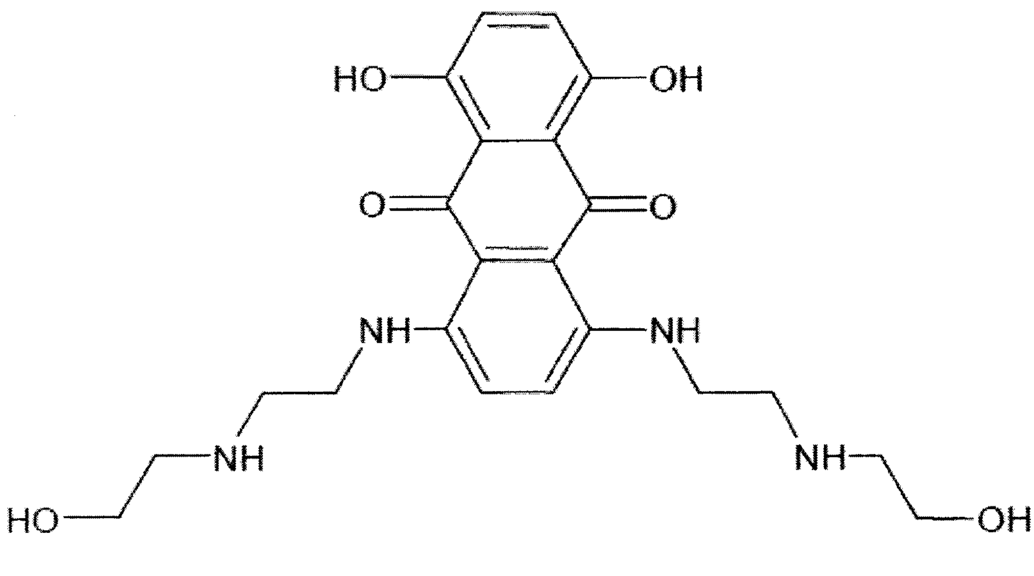

Formelgewicht = 444.48092

Berechnung des Ansatzes:

66000 mg / 3x444,5 mg = 1000 mg / X mg; man erhält für X = 20,2 mg Mitoxantron auf 1g HSA (Humanes Serumalbumin)

Herstellung:

1 g HSA in 50 ml Ampuwa lösen; 20,2 mg Mitoxantron in 10 ml Methanol lösen. Die Methanol Lösung unter Rühren langsam in die HSA-Lösung tropfen lassen. Diese Lösung wird bei -80 °C eingefroren und gefriergetrocknet. Das Lyophilisat wird mit physiologischer Kochsalzlösung resuspendiert. Zur Kontriolle wird ein Aliquot (2 ml) mit Centricon-Ultrafiltration untersucht. Anschließend erfolgt eine Sterilfiltration der Lösung zur Anwendung.

Beispiel 9

Anwendungsbeispiel mTHPC-HSA meso-tetrahydroxyphenylchlorin:

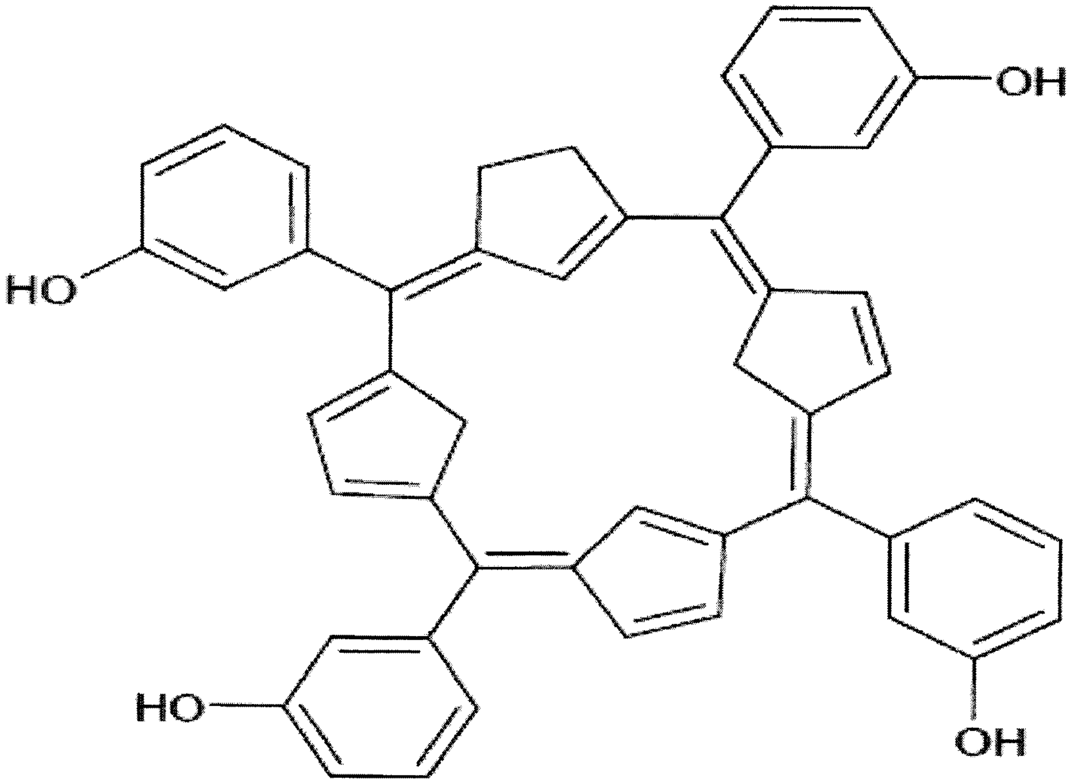

Formelgewicht = 676.79704

Berechnung des Ansatzes:

66000 mg / 3x676,8 mg = 1000 mg / X mg; man erhält für X = 30,7 mg mTHPC auf 1g HSA (Humanes Serumalbumin)

Herstellung:

1 g HSA in 50 ml Ampuwa lösen; 30,7 mg mTHPC in 10ml Ethanol lösen. Die Ethanol Lösung unter Rühren langsam in die HSA-Lösung tropfen lassen. Diese Lösung wird bei -80 °C eingefroren und gefriergetrocknet. Das Lyophilisat wird mit 20 ml physiologischer Kochsalzlösung resuspendiert. Zur Kontrolle wird ein Aliquot (2 ml) mit Centricon-Ultrafiltration untersucht. Anschließend erfolgt eine Sterilfiltration der Lösung zur Anwendung.

meso-tetrahydroxyphenylchlorin = mTHPC = FoscanR

Beispiel 10

mTHPC/HSA

Berechnung des Ansatzes:

66000mg / 676,8mg = 1000mg / X; man erhält für X = 10,2mg mTHPC auf 1g HSA

Herstellung mTHPC-HSA:

1g HSA in 40ml Ampuwa lösen; 10,2mg mTHPC in 4ml Ethanol absolut. lösen. Die Ethanol-Lösung unter Rühren langsam in die HSA-Lösung tropfen lassen. Diese Lösung bei -80° C einfrieren und gefriertrocknen. Das Lyophillisat mit physiologischer Kochsalzlösung wieder auflösen. Anschließend Sterilfiltration der Lösung und Kontrolle des Ansatzes mittels GPC-HPLC.

Beispiel 11

Mitoxantron

Molekularformel = $C_{22}H_{28}N_4O_6$

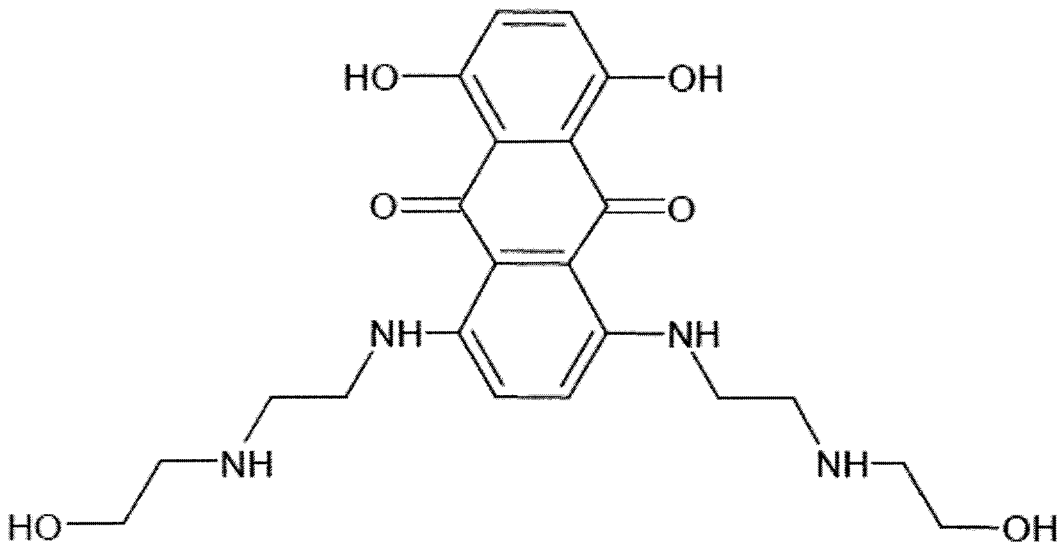

Molekulargewicht = 444.48092

Berechnung des Ansatzes:

66000mg / 444,5mg = 1000mg / X mg; man erhält für X = 7,0 mg Mitoxantron auf 1g HSA

Herstellung:

1g HSA in 30m1 Ampuwa und 0,5 ml Citratpuffer pH 6,0 lösen; 7,0mg Mitoxantron in 3ml Ethanol absolut unter Erwärmung auf 50°C lösen. Die Ethanol Lösung nach Abkühlung auf Raumtemperatur unter Rühren langsam in die HSA-Lösung tropfen lassen. Diese Lösung wird bei -80° C eingefroren und gefriergetrocknet. Das Lyophillisat mit 20 ml physiologischer Kochsalzlösung resuspendiert. Kontrolle eines Aliquotes (2ml) des Ansatzes mittels Centrion-Ultrafiltration oder GPC-HPLC. Anschließend Sterilfiltration der Lösung zur Anwendung.

Beispiel 12

Artemisinin

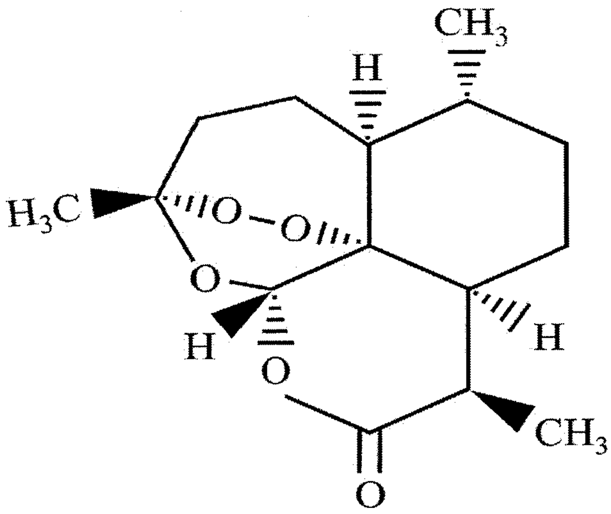

Molekular Gewicht 283 g/Mol

Berechnung des Ansatzes:

66000mg/283mg = 1000mg/X mg ; X = 5,0mg Artemisinin auf 1g HSA

Herstellung:

1g HSA in 50ml Ampuwa lösen; 5mg Artemisinin in 5 ml Ethanol absolut lösen. Die Ethanol-Lösung unter Rühren langsam in die HSA-Lösung tropfen lassen. Diese Lösung wird bei -80° C eingefroren und gefriergetrocknet. Das Lyophillisat mit 20ml physiologischer Kochsalzlösung wieder auflösen. Anschließend Sterilfiltration der Lösung und Kontrolle des Ansatzes mittels GPC-HPLC ( Detektion bei 350nm ).

**Patentansprüche**

1. Verfahren zur Herstellung einer Nanopartikel-freien Wirkstoff-Trägerprotein-Lösung umfassend die Schritte:

   i) Bereitstellen einer wässrigen Trägerprotein-Lösung, wobei als Trägerprotein Serumalbumin eingesetzt wird,
   ii) Auflösen eines schlecht wasserlöslichen oder nicht wasserlöslichen Wirkstoffes in Acetonitril und/oder einem Alkohol ausgewählt aus Methanol, Ethanol, n-Propanol, iso-Propanol und/oder tert-Butanol,
   iii) Zugeben der Wirkstoff-Alkohol-Lösung und/oder der Wirkstoff-Acetonitril-Lösung zu der wässrigen Trägerprotein-Lösung zum Erhalten einer Wirkstoff-Trägerprotein-Lösung, wobei das Molverhältnis von Trägerprotein zu Wirkstoff 10:1 bis 1:3 beträgt und wobei die Wirkstoff-Trägerprotein-Lösung keine Partikel mit einem Durchmesser ≥ 50 nm enthält,

   **dadurch gekennzeichnet, dass**
   der Anteil des Acetonitrils und/oder Alkohols in der in Schritt (iii) erhaltenen Wirkstoff-Trägerprotein-Lösung ≤ 20% v/v ist.

2. Verfahren nach Anspruch 1,
   umfassend die Schritte (iv) und (v) im Anschluss an Schritt (iii):

iv) Lyophilisieren der in Schritt iii) erhaltenen Lösung und
v) Resuspendieren des Lyophilisats zum Wieder-Herstellen der Wirkstoff-Trägerprotein-Lösung.

**3.** Verfahren nach Anspruch 1 oder 2,
**dadurch gekennzeichnet, dass**
als Trägerprotein in Schritt i) humanes oder tierisches Serumalbumin und bevorzugt Liganden-freies Serumalbumin oder rekombinantes humanes Serumalbumin eingesetzt wird.

**4.** Verfahren nach einem der vorhergehenden Ansprüche,
**dadurch gekennzeichnet, dass**
als Wirkstoff Curcumin, Amsacrin, Elipticin, Imatinib, Mitoxantron, Artemisinin, mTHPC oder/und Doxorubicin eingesetzt wird.

**5.** Verfahren nach einem der vorhergehenden Ansprüche,
**dadurch gekennzeichnet, dass**
das Molverhältnis von Trägerprotein zu Wirkstoff 5:1 bis 1:2 beträgt.

**6.** Verfahren nach einem der vorhergehenden Ansprüche,
**dadurch gekennzeichnet, dass**
der Wirkstoff nicht-kovalent an das Trägerprotein gebunden wird.

**7.** Verfahren nach einem der vorhergehenden Ansprüche,
**dadurch gekennzeichnet, dass**
das Trägerprotein in der hergestellten Wirkstoff-Trägerprotein-Lösung in nativer Form vorliegt.

**8.** Verfahren nach einem der vorhergehenden Ansprüche,
**dadurch gekennzeichnet, dass**
das Resuspendieren des Lyophilisats in physiologischer Kochsalzlösung erfolgt.

**9.** Verfahren zur Herstellung eines Nanopartikel-freien Wirkstoff-Trägerprotein-Lyophilisats umfassend die Schritte:

i) Bereitstellen einer wässrigen Trägerprotein-Lösung, wobei als Trägerprotein Serumalbumin eingesetzt wird,
ii) Auflösen eines schlecht wasserlöslichen oder nicht wasserlöslichen Wirkstoffes in Acetonitril und/oder einem Alkohol ausgewählt aus Methanol, Ethanol, n-Propanol, iso-Propanol und/oder tert-Butanol,
iii) Zugeben der Wirkstoff-Alkohol-Lösung und/oder der Wirkstoff-Acetonitril-Lösung zu der wässrigen Trägerprotein- Lösung zum Erhalten einer Wirkstoff-Trägerprotein-Lösung, wobei das Molverhältnis von Trägerprotein zu Wirkstoff 10:1 bis 1:3 beträgt und wobei die Wirkstoff-Trägerprotein-Lösung keine Partikel mit einem Durchmesser ≥ 50 nm enthält,
**dadurch gekennzeichnet, dass**
der Anteil des Acetonitrils und/oder Alkohols in der in Schritt (iii) erhaltenen Wirkstoff-Trägerprotein-Lösung ≤ 20% v/v ist,
iv) Lyophilisieren der in Schritt iii) erhaltenen Lösung.

**10.** Kit, umfassend

i) ein Wirkstoff-Trägerprotein-Lyophilisat erhalten gemäß dem Verfahren nach Anspruch 9 sowie
ii) ein pharmazeutisch annehmbares Medium.

## Claims

**1.** A method for preparing a nanoparticle-free solution of active ingredient and carrier protein comprising the steps of:

i) providing an aqueous solution of carrier protein, wherein serum albumin is used as carrier protein,
ii) dissolving a poorly water-soluble or insoluble active ingredient in acetonitrile and/or alcohol which is selected from methanol, ethanol, n-propanol, iso-propanol and/or tert-butanol,
iii) adding the solution of active ingredient and alcohol and/or the solution of active ingredient and acetonitrile to the aqueous solution of carrier protein for obtaining a solution of active ingredient and carrier protein, wherein

the molar ratio of carrier protein to active ingredient is from 10:1 to 1:3 and, wherein the solution of active ingredient and carrier protein is free of particles having a diameter of ≥ 50 nm,

**characterised in that**
the portion of acetonitrile and/or alcohol in the solution of active ingredient and carrier protein, which is obtained in step (iii), is ≤ 20% v/v.

**2.** Method of claim 1,
comprising the steps (iv) and (v) subsequent to step (iii):

(iv) lyophilize the solution, which is obtained in step (iii), and
(v) resuspend the lyophilisate for recovering the solution of active ingredient and carrier protein.

**3.** Method of claim 1 or 2,
**characterized in that**
in step i) human or animal serum albumin and, preferably, ligand-free serum albumin or recombinant human serum albumin is used as carrier protein.

**4.** Method of any of the preceding claims,
**characterized in that**
curcumin, amsacrine, ellipticine, imatinib, mitoxantrone, artemisinin, mTHCP or/and doxorubicin is used as active ingredient.

**5.** Method of any of the preceding claims,
**characterized in that**
the molar ratio of carrier protein to active ingredient is from 5:1 to 1:2.

**6.** Method of any of the preceding claims,
**characterized in that**
the active ingredient is non-covalently bound to the carrier protein.

**7.** Method of any of the preceding claims,
**characterized in that**
the carrier protein is contained in its native form in said solution of active ingredient and carrier protein.

**8.** Method of any of the preceding claims,
**characterized in that**
the resuspension of the lyophilisate is performed in physiologic saline.

**9.** A method for preparing a nanoparticle-free lyophilisate of active ingredient and carrier protein comprising the steps of:

i) providing an aqueous solution of carrier protein, wherein serum albumin is used as carrier protein,
ii) dissolving a poorly water-soluble or insoluble active ingredient in acetonitrile and/or alcohol which is selected from methanol, ethanol, n-propanol, iso-propanol and/or tert-butanol,
iii) adding the solution of active ingredient and alcohol and/or the solution of active ingredient and acetonitrile to the aqueous solution of carrier protein for obtaining a solution of active ingredient and carrier protein, wherein the molar ratio of carrier protein to active ingredient is from 10:1 to 1:3 and, wherein the solution of active ingredient and carrier protein is free of particles having a diameter of ≥ 50 nm,
**characterised in that**
the portion of acetonitrile and/or alcohol in the solution of active ingredient and carrier protein, which is obtained in step (iii), is ≤ 20% v/v.
(iv) lyophilize the solution, which is obtained in step (iii).

**10.** Kit comprising

i) a lyophilisate of active ingredient and carrier protein, which is obtained according to the method of claim 9 and
ii) a pharmaceutically acceptable medium.

**Revendications**

1. Procédé de préparation d'une solution de principe actif-protéine porteuse exempte de nanoparticules comprenant les étapes consistant à :

   i) fournir une solution de protéine porteuse aqueuse, l'albumine sérique étant utilisée comme protéine porteuse,
   ii) dissoudre un principe actif peu soluble ou insoluble dans l'eau dans de l'acétonitrile et/ou un alcool choisi parmi le méthanol, l'éthanol, le n-propanol, l'iso-propanol et/ou le tert-butanol,
   iii) ajouter la solution de principe actif-alcool et/ou la solution de principe actif-acétonitrile à la solution de protéine porteuse aqueuse pour obtenir une solution de principe actif-protéine porteuse, le rapport molaire de la protéine porteuse au principe actif étant de 10:1 à 1:3 et la solution de principe actif-protéine porteuse ne contenant aucune particule d'un diamètre ≥ 50 nm,

   **caractérisé en ce que**
   la proportion d'acétonitrile et/ou d'alcool dans la solution de principe actif-protéine porteuse obtenue à l'étape (iii) est ≤ 20 % v/v.

2. Procédé selon la revendication 1,
   comprenant les étapes (iv) et (v) suivant l'étape (iii), consistant à :

   iv) lyophiliser la solution obtenue à l'étape iii) et
   v) remettre en suspension le lyophilisat pour restaurer la solution de principe actif-protéine porteuse.

3. Procédé selon la revendication 1 ou 2,
   **caractérisé en ce que**
   de l'albumine sérique humaine ou animale et de préférence de l'albumine sérique exempte de ligand ou de l'albumine sérique humaine recombinante est utilisée comme protéine porteuse à l'étape i).

4. Procédé selon l'une des revendications précédentes,
   **caractérisé en ce que**
   de la curcumine, de l'amsacrine, de l'ellipticine, de l'imatinib, de la mitoxantrone, de l'artémisinine, de la mTHPC et/ou de la doxorubicine sont utilisées comme principe actif.

5. Procédé selon l'une des revendications précédentes,
   **caractérisé en ce que**
   le rapport molaire de la protéine porteuse au principe actif est de 5:1 à 1:2.

6. Procédé selon l'une des revendications précédentes,
   **caractérisé en ce que**
   le principe actif est lié de manière non covalente à la protéine porteuse.

7. Procédé selon l'une des revendications précédentes,
   **caractérisé en ce que**
   la protéine porteuse est présente sous forme native dans la solution de principe actif-protéine porteuse préparée.

8. Procédé selon l'une des revendications précédentes,
   **caractérisé en ce que**
   la remise en suspension du lyophilisat s'effectue dans une solution saline physiologique.

9. Procédé de préparation d'un lyophilisat de principe actif-protéine porteuse exempt de nanoparticules, comprenant les étapes consistant à :

   i) fournir une solution de protéine porteuse aqueuse, l'albumine sérique étant utilisée comme protéine porteuse,
   ii) dissoudre un principe actif peu soluble ou insoluble dans l'eau dans de l'acétonitrile et/ou un alcool choisi parmi le méthanol, l'éthanol, le n-propanol, l'iso-propanol et/ou le tert-butanol,
   iii) ajouter la solution de principe actif-alcool et/ou la solution de principe actif-acétonitrile à la solution de protéine porteuse aqueuse pour obtenir une solution de principe actif-protéine porteuse, le rapport molaire de la protéine porteuse au principe actif étant de 10:1 à 1:3 et la solution de principe actif-protéine porteuse ne contenant

aucune particule d'un diamètre ≥ 50 nm,
**caractérisé en ce que**
la proportion d'acétonitrile et/ou d'alcool dans la solution de principe actif-protéine porteuse obtenue à l'étape (iii) est ≤ 20 % v/v,
iv) lyophiliser la solution obtenue à l'étape iii).

**10.** Kit, comprenant

i) un lyophilisat de principe actif-protéine porteuse obtenu conformément au procédé selon la revendication 9 et
ii) un milieu pharmaceutiquement acceptable.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19636889 A1
- WO 0071079 A2
- DE 19847362 A1
- US 2011142948 A1
- CN 103054810 A1
- CN 102512404 B


### In der Beschreibung aufgeführte Nicht-Patentliteratur


- **K. SANTHI et al.** *Drug Development and Industrial Pharmacy,* 2002, vol. 28 (9), 1171-1179
- **Z. SONG et al.** *Drug Design, Development and Therapy,* 2016, vol. 10, 2643-2649
- **S. YAN et al.** *Procedia Engineering,* 2015, vol. 102, 590-601